# EUROPÄISCHE PATENTSCHRIFT

(11) **EP 3 744 416 B1**
(45) Veröffentlichungstag und Bekanntmachung des Hinweises auf die Patenterteilung: **24.11.2021**
(21) Anmeldenummer: 19180022.6
(22) Anmeldetag: 13.06.2019
(51) Int. Cl.: B01D 53/22, C07C 31/04, C07C 29/151, C07C 29/15, C01B 3/00, C01B 3/36, C01B 3/38, C01B 3/56

(54) **VERFAHREN UND ANLAGE ZUR SYNTHESE VON METHANOL**
METHOD AND SYSTEM FOR SYNTHESISING METHANOL
PROCÉDÉ ET INSTALLATION DE SYNTHÈSE DE MÉTHANOL

(30) Priorität: 28.05.2019 EP 19177102
(43) Veröffentlichungstag der Anmeldung: 02.12.2020
(73) Patentinhaber: thyssenkrupp Industrial Solutions AG, 45143 Essen (DE); GasConTec GmbH, 61348 Bad Homburg v. d. Höhe (DE)
(72) Erfinder: SCHULZ, Alexander, 60439 Frankfurt (DE)
(74) Vertreter: Patentanwälte Bauer Vorberg Kayser

(56) Entgegenhaltungen:
- EP-A1- 2 011 564
- WO-A1-2005/108336

## Beschreibung

Die Erfindung betrifft ein Verfahren zur Synthese von Methanol gemäß dem Oberbegriff von Anspruch 1 sowie eine Anlage zur Synthese von Methanol gemäß dem Oberbegriff von Anspruch 16.

Die Herstellung von Methanol findet regelmäßig in einem Reaktor einer Anlage für die Synthese von Methanol statt, welchem Reaktor ein Synthesegasstrom mit Wasserstoff und Kohlenstoffoxiden zugeführt wird und in welchem die exotherme Reaktion zur Herstellung von Methanol abläuft.

Abhängig von der Art und Weise, wie der Synthesegasstrom gewonnen wird und abhängig davon, welcher Energieträger die Grundlage für das Synthesegas bildet, kann der Anteil des Wasserstoffs in dem frischen Synthesegas niedriger sein als an sich angestrebt. Insbesondere zur Verbesserung der Stöchiometrie bei der Methanolsynthese ist es daher häufig zweckmäßig, aus einem Restgas des Reaktors unreagierten Wasserstoff zurückzugewinnen und diesen Wasserstoff zum Methanolreaktor zurückzuführen.

Für eine solche Rückführung ist es erforderlich, den Druck des zurückgewonnenen Wasserstoffs vor Zuführung zu dem Reaktor zu erhöhen.

Die EP 3 205 622 B1 aus dem Stand der Technik, von welcher die vorliegende Erfindung als nächstkommend ausgeht, offenbart eine Anlage zur Synthese von Methanol. Bei dieser Anlage wird ein Wasserstoffstrom, welcher mittels einer PSA aus einem Restgas der Methanolsynthese gewonnen wurde, dem Synthesegasstrom zugeführt. Diese Zuführung erfolgt dem Synthesegaskompressor prozesstechnisch vorgelagert, sodass also der Wasserstoff aus dem Wasserstoffstrom gemeinsam mit dem Synthesegas durch den Synthesegaskompressor vor Zuführung zu dem Methanolreaktor druckerhöht wird. Die EP 2011564 A1 offenbart eine Anlage und Herstellung zur Synthese von Methanol.

Nachteilig an diesem Stand der Technik ist, dass der Synthesegaskompressor durch das Erfordernis, auch den rückgewonnenen Wasserstoff zu komprimieren, größer als zuvor dimensioniert werden muss und ebenso der Energieverbrauch für die Druckerhöhung steigt.

Ausgehend von diesem Stand der Technik besteht die Aufgabe der Erfindung daher darin, das aus dem Stand der Technik bekannte Verfahren zur Synthese von Methanol dahingehend zu verbessern, dass eine wirtschaftlich günstigere Druckerhöhung des zurückgewonnenen Wasserstoffs erreicht werden kann.

Bezogen auf ein Verfahren zur Synthese von Methanol gemäß dem Oberbegriff von Anspruch 1 wird diese Aufgabe durch die Merkmale des kennzeichnenden Teils von Anspruch 1 gelöst. Bezogen auf eine Anlage zur Synthese von Methanol gemäß dem Oberbegriff von Anspruch 16 wird diese Aufgabe durch die Merkmale des kennzeichnenden Teils von Anspruch 16 gelöst.

Der Erfindung liegt die Erkenntnis zugrunde, dass eine Druckerhöhung des extrahierten Wasserstoffs aus der Wasserstoffrückgewinnung auch durch den Recyclekompressor durchgeführt werden kann. Dieser Recyclekompressor dient dazu, den wesentlichen Teil des unreagierten Restgases durch den Methanolreaktor zu zirkulieren. Regelmäßig wird nur ein Teilstrom des unreagierten Restgases der Wasserstoffrückgewinnung zugeführt, da dann regelmäßig dieser gesamte Teilstrom - abzüglich des rückgewonnenen Wasserstoffs - als Purgegas abgeführt werden kann. Soll der rückgewonnene Wasserstoff dann wieder mit dem übrigen unreagierten Restgas zwecks Druckerhöhung durch den Recyclekompressor zusammengeführt werden, so ist zwar unter Umständen ein gewisser Druckverlust dieses übrigen unreagierten Restgases erforderlich, da die Wasserstoffrückgewinnung ebenfalls mit einem bestimmten Druckverlust einhergeht. Es hat sich aber herausgestellt, dass dennoch die Verlagerung der Druckerhöhung des rückgewonnenen Wasserstoffs von dem Synthesegaskompressor zu dem Recyclekompressor im Ergebnis zu niedrigeren Kosten führt.

Das vorschlagsgemäße Verfahren dient der Synthese von Methanol. Bei dem vorschlagsgemäßen Verfahren wird ein kohlenstoffhaltiger Energieträgerstrom einer Synthesegasreaktoranordnung zum Gewinnen eines Synthesegasstroms mit Wasserstoff und Kohlenstoffoxiden zugeführt. Der Synthesegasstrom weist also Wasserstoff, Kohlenstoffmonoxid und Kohlenstoffdioxid auf und kann daneben noch weitere Bestandteile wie insbesondere Stickstoff und Edelgase aufweisen. Der Synthesegasstrom kann auch als Frischgasstrom bezeichnet werden.

Ebenso wird bei dem vorschlagsgemäßen Verfahren der Synthesegasstrom einer Wärmerückgewinnungsvorrichtung zum Rückgewinnen von Wärme aus dem Synthesegasstrom und danach einem Synthesegaskompressor zur Druckerhöhung zugeführt. Dieser Synthesegaskompressor kann mehrstufig ausgestaltet sein. Denkbar ist, dass der Synthesegasstrom zwischen der Wärmerückgewinnungsvorrichtung und dem Synthesegaskompressor einer anderen Vorrichtung oder mehreren anderen Vorrichtungen zugeführt wird. Weiter ist zu beachten, dass die Wärmerückgewinnungsvorrichtung regelmäßig nur eine Stufe einer Wärmerückgewinnungsanordnung mit mehreren Wärmerückgewinnungsvorrichtungen darstellt. Anders ausgedrückt kann es sein, dass der Synthesegasstrom nur einer Wärmerückgewinnungsvorrichtung von mehreren, miteinander zusammenhängenden Wärmerückgewinnungsvorrichtungen zugeführt wird.

Gemäß dem vorschlagsgemäßen Verfahren wird der druckerhöhte Synthesegasstrom zumindest teilweise einer ersten Reaktorstufe einer Methanol-Reaktoranordnung zur teilweisen Umwandlung in Methanol zugeführt. Bevorzugt ist, dass der druckerhöhte Synthesegasstrom im Wesentlichen vollständig der ersten Reaktorstufe zugeführt wird. Es kann aber auch sein, dass ein Teil des Synthesegasstroms vorher abgezweigt wird. Das Merkmal der teilweisen Umwandlung in Methanol begründet sich darin, dass ein nicht umgesetzter Rest an Edukten aus der Methanol-Reaktoranordnung austritt und daher die Umwandlung nicht vollständig abläuft. Die Methanol-Reaktoranordnung kann mehrere Reaktorstufen oder nur eine einzelne Reaktorstufe aufweisen. Weist die Methanol-Reaktoranordnung nicht mehrere Reaktorstufen auf, so handelt es sich bei der ersten Reaktorstufe um die einzige Reaktorstufe der Methanol-Reaktoranordnung. Die erste Reaktorstufe der Methanol-Reaktoranordnung ist diejenige Reaktorstufe der Methanol-Reaktoranordnung, welcher der Synthesegasstrom zumindest teilweise zugeführt wird, bevor er oder ein verbliebener Restgasstrom einer weiteren Reaktorstufe zugeführt wird. Die erste Reaktorstufe ist insoweit die prozesstechnisch zuerst gelagerte Reaktorstufe der Methanol-Reaktoranordnung. Dieser Umstand deckt sich mit der möglichen Bezeichnung des Synthesegasstroms als Frischgasstrom. Jede einzelne Reaktorstufe der Methanol-Reaktoranordnung kann dabei mehrere, prozesstechnisch zueinander parallel geschaltete Einzelreaktoren für die Methanolsynthese aufweisen.

Beim vorschlagsgemäßen Verfahren ist vorgesehen, dass aus der Methanol-Reaktoranordnung ein Restgasstrom mit unreagierten Kohlenstoffoxiden gewonnen wird, welcher Restgasstrom einem Recyclekompressor zur Druckerhöhung des Restgasstroms zugeführt wird. Der Restgasstrom kann auch unreagierten Wasserstoff aufweisen. Sollte die Methanol-Reaktoranordnung mehr als eine Reaktorstufe aufweisen, so kann dieser Restgasstrom nach einer beliebigen Reaktorstufe gewonnen werden. Unter einem unreagierten Stoff ist hier und nachfolgend ein Stoff zu verstehen, welcher als Edukt für die Methanolsynthese einer Reaktorstufe der Methanol-Reaktoranordnung, insbesondere der ersten Reaktorstufe, zugeführt wurde und dann aus der Reaktorstufe ausgetreten ist, ohne an einer Reaktion zur Synthese von Methanol teilgenommen zu haben.

Das vorschlagsgemäße Verfahren sieht weiter vor, dass der druckerhöhte Restgasstrom der Methanol-Reaktoranordnung zur teilweisen Umwandlung in Methanol zugeführt wird. Es handelt sich also um eine Rückführung des nun druckerhöhten Restgasstroms zur Methanol-Reaktoranordnung, aus welcher der Restgasstrom ja gewonnen wurde.

Das vorschlagsgemäße Verfahren sieht zusätzlich vor, dass ein Rückgewinnungsstrom aus einem unreagierten Restgas der ersten Reaktorstufe einer Wasserstoffrückgewinnungsanordnung zum Gewinnen eines H-Recyclestroms zugeführt wird. Bei dem unreagierten Restgas kann es sein, dass es sich nur um einen Teil des gesamten unreagierten Gases aus der ersten Reaktorstufe handeln. Der Rückgewinnungsstrom kann aus dem unreagierten Restgas etwa durch Abzweigen gewonnen werden.

Das vorschlagsgemäße Verfahren sieht vor, dass der H-Recyclestrom unreagierten Wasserstoff aus dem unreagierten Restgas aufweist, welcher unreagierte Wasserstoff des H-Recyclestroms erneut der ersten Reaktorstufe zur zumindest teilweisen Umwandlung in Methanol zugeführt wird. Es kann sein, dass der unreagierte Wasserstoff nur ein Teil des gesamten unreagierten Wasserstoffs der ersten Reaktorstufe und/oder nur ein Teil des gesamten unreagierten Wasserstoffs des unreagierten Restgases ist. Die erneute Zuführung des unreagierten Wasserstoffes des H-Recyclestroms zur ersten Reaktorstufe kann dabei sowohl direkt als auch indirekt erfolgen. In dem Fall der indirekten Zuführung wird der unreagierte Wasserstoff also zunächst anderen Vorrichtungen zugeführt.

Das vorschlagsgemäße Verfahren ist dadurch gekennzeichnet, dass der unreagierte Wasserstoff des H-Recyclestroms zumindest teilweise von der ersten Reaktorstufe bis zur erneuten Zuführung zur ersten Reaktorstufe durch den Recyclekompressor mit den unreagierten Kohlenstoffoxiden druckerhöht wird. Anders ausgedrückt findet zumindest für einen Teil des unreagierten Wasserstoffs im H-Recyclestrom zwischen dem Austritt dieses unreagierten Wasserstoffes aus der ersten Reaktorstufe und der erneuten Zuführung dieses unreagierten Wasserstoffes zu der ersten Reaktorstufe eine Druckerhöhung durch den Recyclekompressor statt. Da der Recyclekompressor - wie bereits festgestellt - den Restgasstrom mit unreagierten Kohlenstoffoxiden druckerhöht, findet also diese Druckerhöhung dieses unreagierten Wasserstoffs durch den Recyclekompressor zusammen mit den unreagierten Kohlenstoffoxiden statt. Bevorzugt ist, dass der unreagierte Wasserstoff des H-Recyclestroms im Wesentlichen vollständig von der ersten Reaktorstufe bis zur erneuten Zuführung zur ersten Reaktorstufe durch den Recyclekompressor mit den unreagierten Kohlenstoffoxiden druckerhöht wird.

Grundsätzlich kann der unreagierte Wasserstoff des H-Recyclestroms oder ein Teil dieses Wasserstoffs auch mehrmals vor der erneuten Zuführung zur ersten Reaktorstufe druckerhöht werden. So kann einerseits neben der Druckerhöhung durch den Recyclekompressor zusätzlich eine Druckerhöhung durch den Synthesegaskompressor erfolgen. Es kann sein, dass diese zusätzliche Druckerhöhung nur einen Teil des unreagierten Wasserstoffs betrifft. Andererseits kann es auch sein, dass der unreagierte Wasserstoff oder ein Teil davon mehrmals durch den Recyclekompressor vor der erneuten Zuführung zu der ersten Reaktorstufe druckerhöht wird. Das kann insbesondere dann der Fall sein, wenn ausgangsseitig zum Recyclekompressor eine Rückführung zur Eingangsseite des Recyclekompressors erfolgt.

Eine bevorzugte Ausführungsform des Verfahrens ist jedoch dadurch gekennzeichnet, dass der unreagierte Wasserstoff des H-Recyclestroms zumindest teilweise, vorzugsweise im Wesentlichen vollständig, von der ersten Reaktorstufe bis zur erneuten Zuführung zur ersten Reaktorstufe durch den Recyclekompressor mit den unreagierten Kohlenstoffoxiden genau einmal druckerhöht wird. Folglich wird zumindest ein Teil des unreagierten Wasserstoffs des H-Recyclestroms von der ersten Reaktorstufe bis zur erneuten Zuführung zur ersten Reaktorstufe nur durch den Recyclekompressor mit den unreagierten Kohlenstoffoxiden druckerhöht. Es findet daher auch keine Druckerhöhung durch einen anderen Kompressor o.dgl. statt. An sich überflüssige wiederholte Druckerhöhungen werden damit vermieden. Zu beachten ist, dass dieses Erfordernis der genau einmaligen Druckerhöhung durch den Recyclekompressor nur den unreagierten Wasserstoff aus der ersten Reaktorstufe betrifft, welcher auch im H-Recyclestrom enthalten ist und auch von diesem unreagierten Wasserstoff vorzugsweise nur einen Teil. Sollte es also - wie regelmäßig der Fall ist - unreagierten Wasserstoff aus der ersten Reaktorstufe geben, welcher nicht in dem H-Recyclestrom enthalten ist, so ist es nicht erforderlich, dass auch dieser unreagierte Wasserstoff außerhalb des Rückgewinnungsstroms genau eine Druckerhöhung durch den Recyclekompressor erfährt. Vielmehr ist dann insbesondere auch eine mehrfache Druckerhöhung möglich.

Wie untenstehend noch beschrieben wird, kann diese erneute Zuführung des unreagierten Wasserstoffs zu der ersten Reaktorstufe indirekt derart erfolgen, dass der Wasserstoff als Teil einer Reihe weiterer Ströme der ersten Reaktorstufe zugeführt wird.

Grundsätzlich kann es sich bei der obigen Druckerhöhung des unreagierten Wasserstoffs um eine Druckerhöhung um einen beliebigen Betrag handeln. Bevorzugt ist, dass der unreagierte Wasserstoff vor der zumindest teilweisen Umwandlung in Methanol auf einen Druck erhöht wird, welcher höher als der Druck des H-Recyclestroms aus der Wasserstoffrückgewinnungsanordnung ist. Ebenso kann es sein, dass der unreagierte Wasserstoff vor der erneuten Zuführung zur ersten Reaktorstufe auf einen Druck erhöht wird, welcher höher als der Druck des Rückgewinnungsstroms bei Zuführung zu der Wasserstoffrückgewinnungsanordnung ist.

Die Druckerhöhung des unreagierten Wasserstoffs kann einerseits vor der Zuführung zur Wasserstoffrückgewinnungsanordnung erfolgen. So kann es sein, dass der Rückgewinnungsstrom insgesamt druckerhöht wird. Die Druckerhöhung des unreagierten Wasserstoffs kann aber auch nach Zuführung zur Wasserstoffrückgewinnungsanordnung erfolgen. Daher kann etwa die Druckerhöhung des unreagierten Wasserstoffs des H-Recyclestroms dadurch erfolgen, dass der H-Recyclestrom insgesamt druckerhöht wird.

Die Synthesegasreaktoranordnung, der Synthesegaskompressor, die Methanol-Reaktoranordnung, die Wärmerückgewinnungsvorrichtung, der Recyclekompressor und die Wasserstoffrückgewinnungsanordnung können von einer Anlage zur Synthese von Methanol umfasst sein.

Vorzugsweise weist der aus der Synthesegasreaktoranordnung gewonnene Synthesegasstrom einen Erzeugungsdruck auf, welcher mehr als 40 bar, vorzugsweise mehr als 50 bar, insbesondere mehr als 60 bar, weiter insbesondere mehr als 70 bar beträgt.

Eine bevorzugte Ausführungsform des Verfahrens ist dadurch gekennzeichnet, dass der druckerhöhte Synthesegasstrom einen Druck von mehr als 60 bar, vorzugsweise von mehr als 70 bar, insbesondere von mehr als 80 bar und weiter vorzugsweise von mehr als 90 bar.

Grundsätzlich kann der dem Recyclekompressor zugeführte Restgasstrom eine beliebige Zusammensetzung aufweisen, solange der Restgasstrom unreagierte Kohlenstoffoxide zu einem grundsätzlich beliebigen Anteil und den unreagierten Wasserstoff des Rückgewinnungsstroms umfasst. Bevorzugt ist jedoch, dass der dem Recyclekompressor zugeführte Restgasstrom einen molaren Wasserstoffanteil kleiner als 90 %, insbesondere kleiner als 85 % und weiter insbesondere kleiner als 80 % aufweist. Alternativ oder zusätzlich kann es sein, dass der dem Recyclekompressor zugeführte Restgasstrom einen molaren Wasserstoffanteil von größer als 50 %, insbesondere von größer als 60 % und weiter insbesondere von größer als 70 % aufweist. Dieser molare Wasserstoffanteil bezieht sich auf den gesamten molaren Wasserstoffanteil des Restgasstroms. Daher zählt nicht nur der unreagierte Wasserstoff aus dem H-Recyclestrom dazu, sondern auch sonstiger Wasserstoff in dem Restgasstrom.

Vorzugsweise umfasst die Methanol-Reaktoranordnung eine Methanol-Trennvorrichtung zum Gewinnen des unreagierten Restgases der ersten Reaktorstufe und eines Rohmethanolstroms der ersten Reaktorstufe. Grundsätzlich kann die Methanol-Trennvorrichtung auf beliebige Art und Weise funktionieren. Insbesondere kann es sein, dass die Methanol-Trennvorrichtung eine Kondensationsvorrichtung zum Gewinnen des unreagierten Restgases der ersten Reaktorstufe und des Rohmethanolstroms der ersten Reaktorstufe durch Kondensation umfasst.

Es kann sein, dass nur ein Teil des druckerhöhten Restgasstroms der Methanol-Reaktoranordnung zugeführt wird. Insbesondere ist es bevorzugt, dass ein Teil des druckerhöhten Restgasstroms abgezweigt und der Synthesegasreaktoranordnung zugeführt wird. Dabei kann insbesondere vorgesehen sein, dass der abgezweigte Teil des druckerhöhten Restgasstroms dem Energieträgerstrom zugeführt wird.

Wie bereits festgestellt kann es prinzipiell sein, dass die Methanol-Reaktoranordnung nur eine einzige Methanol-Reaktorstufe umfasst. Eine weitere bevorzugte Ausführungsform des Verfahrens ist dadurch gekennzeichnet, dass die Methanol-Reaktoranordnung eine Vielzahl von prozesstechnisch hintereinander geschalteten Reaktorstufen zur Methanolsynthese aufweist. Jede einzelne Reaktorstufe kann dabei einen oder mehrere Reaktoren aufweisen. Die Reaktoren einer Reaktorstufe können dabei insbesondere untereinander prozesstechnisch parallel angeordnet sein. Weiter kann es sein, dass durch die Methanol-Trennvorrichtung ein jeweiliges unreagiertes Restgas aus jeder der Vielzahl von Reaktorstufen gewonnen wird.

Dass die Reaktorstufen prozesstechnisch hintereinandergeschaltet sind bedeutet, dass Restgas aus einer Reaktorstufe - sofern es nicht die letzte Reaktorstufe in der Reihe der Reaktorstufen ist - direkt oder indirekt der jeweils danach geschalteten Reaktorstufe zugeführt wird. Grundsätzlich kann der obige Recyclekompressor bezüglich der Vielzahl von Reaktorstufen beliebig angeordnet sein. Eine Variante ist, dass der Recyclekompressor prozesstechnisch zwischen zwei Reaktorstufen angeordnet ist. Das bedeutet, dass dem Recyclekompressor zumindest ein Teil des unreagierten Restgases aus einer Reaktorstufe als Restgasstrom zugeführt wird und der druckerhöhte Restgasstrom dann der dieser Reaktorstufe nachgelagerten Reaktorstufe zugeführt wird.

Grundsätzlich kann der H-Recyclestrom beliebig geführt werden, solange mindestens ein Teil seines Wasserstoffs in Methanol umgewandelt wird. Gemäß einer weiteren bevorzugten Ausführungsform des Verfahrens ist diesbezüglich bevorzugt, dass der H-Recyclestrom dem unreagierten Restgas einer prozesstechnisch der ersten Reaktorstufe nachgelagerten Reaktorstufe zugeführt wird. Mit anderen Worten wird der unreagierte Wasserstoff des H-Recyclestroms nach der Zuführung gemeinsam mit zumindest einem Teil des unreagierten Restgases einer anderen Reaktorstufe als der ersten Reaktorstufe behandelt. Auf diese Weise "überspringt" der H-Recyclestrom eine oder mehrere Reaktorstufen nach der ersten Reaktorstufe. Der Vorteil eines solchen Ansatzes liegt darin, dass auf diese Weise der Druckverlust des H-Recyclestroms durch die Wasserstoffrückgewinnung im Grunde parallel zu dem Druckverlust des unreagierten Restgases der nachgelagerten Reaktorstufe in dieser Reaktorstufe anfällt. Anders ausgedrückt liegt dadurch der jeweilige Druck dieses unreagierten Restgases und des H-Recyclestroms näher beieinander, was wiederum einen beim Zusammenführen durch das Angleichen auf das niedrigere Druckniveau entstehenden Druckverlust verringert.

Bevorzugt ist, dass der H-Recyclestrom dem Recyclekompressor zur Druckerhöhung mit dem Restgasstrom zugeführt wird.

Gemäß einer bevorzugten Ausführungsform des Verfahrens ist vorgesehen, dass der Restgasstrom aus einer prozesstechnisch der ersten Reaktorstufe nachgelagerten Reaktorstufe gewonnen wird. Mit anderen Worten entstammt dann der dem Recyclekompressor zugeführte Restgasstrom nicht der ersten Reaktorstufe - also der Reaktorstufe, welcher der Synthesegasstrom zumindest teilweise unmittelbar zugeführt wird - sondern einer nachgelagerten Reaktorstufe. Weiter kann es sein, dass der Recyclekompressor den druckerhöhten Restgasstrom der ersten Reaktorstufe zuführt. Grundsätzlich kann der druckerhöhte Restgasstrom aber auch einer anderen Reaktorstufe der Vielzahl von Reaktorstufen zugeführt werden. Ebenso kann es sein, dass der druckerhöhte Restgasstrom aufgeteilt und mehreren Reaktorstufen der Vielzahl von Reaktorstufen zugeführt wird.

Eine weitere bevorzugte Ausführungsform des vorschlagsgemäßen Verfahrens ist dadurch gekennzeichnet, dass der Restgasstrom aus einer prozesstechnisch zuletztgelagerten Reaktorstufe der Vielzahl von Reaktorstufen gewonnen wird. Diese und die vorangehende Variante erlaubt es ebenfalls, für ein Zusammenführen von Strömen erforderliche Druckverluste zu verringern.

Grundsätzlich kann der Rückgewinnungsstrom an einer beliebigen Stelle und aus einem beliebigen Ursprung innerhalb der Methanol-Reaktoranordnung gewonnen werden. Der Rückgewinnungsstrom enthält unreagierten Wasserstoff aus einem unreagierten Restgas der ersten Reaktorstufe. Eine erste bevorzugte Variante sieht vor, dass der Rückgewinnungsstrom zumindest teilweise aus dem unreagierten Restgas der ersten Reaktorstufe abgezweigt wird. Es kann sein, dass der Rückgewinnungsstrom zumindest teilweise dem Recyclekompressor prozesstechnisch vorgelagert abgezweigt wird.

Es kann aber auch sein, dass der Rückgewinnungsstrom bereits eine Druckerhöhung durch den Recyclekompressor erfahren hat. Eine weitere bevorzugte Ausführungsform des Verfahrens ist daher dadurch gekennzeichnet, dass der Rückgewinnungsstrom der Wasserstoffrückgewinnungsanordnung mit einem Zuführungsdruck zugeführt wird, welcher höher ist als ein Restgasdruck, mit welchem der Restgasstrom aus der Methanol-Reaktoranordnung gewonnen wird. Eine bevorzugte Möglichkeit zur Erhöhung des Drucks des Rückgewinnungsstroms besteht darin, diesen zuvor durch den Recyclekompressor komprimieren zu lassen. Entsprechend ist es bevorzugt, dass der Rückgewinnungsstrom zumindest teilweise dem Recyclekompressor prozesstechnisch nachgelagert aus dem Restgasstrom abgezweigt wird.

Es kann aber auch sein, dass der Wasserstoffrückgewinnungsanordnung mehr als ein Strom zugeführt wird, aus dem Wasserstoff gewonnen wird. Gemäß einer bevorzugten Ausführungsform des Verfahrens ist so etwa vorgesehen, dass zumindest ein Teil des insbesondere druckerhöhten Synthesegasstroms für die Zuführung an eine Wassergas-Shift-Reaktionsvorrichtung abgezweigt wird. Es kann auch der vorzugsweise druckerhöhte Synthesegasstrom insgesamt der Wassergas-Shift-Reaktionsvorrichtung zugeführt werden. Ebenso ist es bevorzugt, dass ein weiterer Rückgewinnungsstrom zumindest teilweise aus der Wassergas-Shift-Reaktionsvorrichtung gewonnen und der Wasserstoffrückgewinnungsanordnung zum Gewinnen des H-Recyclestroms zugeführt wird. Mit anderen Worten wird zumindest ein Teil des Wasserstoffs des H-Recyclestroms aus diesem weiteren Rückgewinnungsstrom gewonnen. Diese Wassergas-Shift-Reaktionsvorrichtung kann von der Anlage zur Synthese von Methanol umfasst sein.

Insbesondere kann in der Wassergas-Shift-Reaktionsvorrichtung durch eine Wassergas-Shift-Reaktion zumindest ein Teil des Kohlenstoffoxids im insbesondere druckerhöhten Synthesegasstrom zu Kohlenstoffdioxid und Wasserstoff reagieren. Durch die Erhöhung des Wasserstoffanteils kann somit die Stöchiometrie für die Methanolsynthese verbessert werden.

Ebenso kann es grundsätzlich sein, dass der H-Recyclestrom zunächst nicht der ersten Reaktorstufe zugeführt wird. Gemäß einer weiteren bevorzugten Ausführungsform des Verfahrens ist hingegen vorgesehen, dass der H-Recyclestrom dem insbesondere druckerhöhten Synthesegasstrom zugeführt wird. Das bedeutet speziell, dass der H-Recyclestrom dem Synthesegasstrom der Synthesegasreaktoranordnung prozesstechnisch nachgelagert zugeführt wird. Vorzugsweise wird der H-Recyclestrom dem Synthesegaskompressor prozesstechnisch nachgelagert dem Synthesegasstrom zugeführt. Anders ausgedrückt wird der H-Recyclestrom dem Synthesegasstrom der ersten Reaktorstufe prozesstechnisch vorgelagert zugeführt. Durch die Zuführung des Synthesegasstroms zur ersten Reaktorstufe der Methanol-Reaktoranordnung wird der Wasserstoff des H-Recyclestroms im Ergebnis aber wieder der ersten Reaktorstufe zugeführt.

Die Synthesegasreaktoranordnung kann neben einem Reaktor zur Erzeugung des Synthesegases weitere Vorrichtungen aufweisen. So kann die Synthesegasreaktoranordnung eine jeweils dem Reaktor prozesstechnisch vorgelagerte Vorrichtung zur Entschwefelung des kohlenstoffhaltigen Energieträgerstroms, eine Sättigungsstufe zur Sättigung des kohlenstoffhaltigen Energieträgerstroms mit Wasser, einen Pre-Reformer zur Vorreformierung des kohlenstoffhaltigen Energieträgerstroms und/oder eine Vorrichtung zum Aufheizen des kohlenstoffhaltigen Energieträgerstroms aufweisen.

Grundsätzlich kann das Gewinnen des Synthesegasstroms aus dem Energieträgerstrom auf beliebige Art und Weise erfolgen. Bevorzugt ist, dass zum Gewinnen des Synthesegasstroms ein sauerstoffhaltiger Strom der Synthesegasreaktoranordnung zugeführt wird. Grundsätzlich kann der sauerstoffhaltige Strom neben dem Sauerstoff noch weitere Bestandteile aufweisen. So kann es sich bei dem sauerstoffhaltigen Strom auch um Umgebungsluft handeln.

Grundsätzlich kann der Synthesegasstrom etwa durch eine Dampfreformierung des kohlenstoffhaltigen Energieträgerstroms gewonnen werden. Eine weitere bevorzugte Ausführungsform des Verfahrens ist dadurch gekennzeichnet, dass in der Synthesegasreaktoranordnung der Synthesegasstrom durch eine autotherme Reformierung aus dem kohlenstoffhaltigen Energieträgerstrom gewonnen wird. Bei einer solchen autothermen Reformierung stellt eine katalytische partielle Oxidation die für die endothermen Reformierungsreaktionen erforderliche Wärme bereit. Gegenüber einer reinen Dampfreformierung bietet die autotherme Reformierung den Vorteil, dass der Synthesegasstrom mit einem höheren Druck bereitgestellt werden kann. Alternativ oder zusätzlich kann es sein, dass in der Synthesegasreaktoranordnung der Synthesegasstrom durch eine partielle Oxidation aus dem kohlenstoffhaltigen Energieträgerstrom gewonnen wird.

Grundsätzlich kann eine autotherme Reformierung auch mit Umgebungsluft betrieben werden. Bevorzugt ist jedoch, dass der sauerstoffhaltige Strom aus einer Luftzerlegungsvorrichtung zum Gewinnen eines Sauerstoffstroms aus einer Umgebungsluft gewonnen wird. Die Luftzerlegungsvorrichtung kann darüber hinaus auch zum Gewinnen eines Stickstoffstroms eingerichtet sein. Insbesondere kann es dann sein, dass der sauerstoffhaltige Strom im Wesentlichen aus Sauerstoff besteht. Auf diese Weise wird der Anteil an inerten Gasen bei der Methanolsynthese verringert, sodass verschiedene Vorrichtungen der Anlage kleiner dimensioniert werden können. Vorzugsweise umfasst die Anlage zur Synthese von Methanol die Luftzerlegungsvorrichtung.

Gemäß einer bevorzugten Ausführungsform des Verfahrens ist vorgesehen, dass der H-Recyclestrom dem Energieträgerstrom zugeführt wird. Insbesondere kann es sein, dass der H-Recyclestrom dem Energieträgerstrom der Synthesegasreaktoranordnung prozesstechnisch vorgelagert zugeführt wird.

Neben dem H-Recyclestrom kann die Wasserstoffrückgewinnungsanordnung auch weitere Ströme ausgeben. Vorzugsweise gibt die Wasserstoffrückgewinnungsanordnung einen Purgestrom aus. Dieser kann insbesondere zur Verfeuerung abgeführt werden.

Grundsätzlich kann der H-Recyclestrom eine beliebige Zusammensetzung aufweisen, sofern er den unreagierten Wasserstoff aus dem unreagierten Restgas der ersten Reaktorstufe enthält. Gemäß einer weiteren bevorzugten Ausführungsform des Verfahrens ist vorgesehen, dass der H-Recyclestrom einen höheren molaren Anteil an Wasserstoff als der Rückgewinnungsstrom aufweist. Dies bezieht sich nicht nur auf den unreagierten Wasserstoff aus dem unreagierten Restgas der ersten Reaktorstufe, sondern auf den Wasserstoff im H-Recyclestrom insgesamt. Mit anderen Worten ist in dem H-Recyclestrom der Wasserstoff gegenüber dem Rückgewinnungsstrom angereichert. Ebenso ist es bevorzugt, dass der H-Recyclestrom einen höheren molaren Anteil an Wasserstoff als der Purgestrom aufweist.

Grundsätzlich kann die Wasserstoffrückgewinnungsanordnung nach einem beliebigen Prinzip funktionieren, so etwa basierend auf einer Membran oder einer Kältevorrichtung. Eine bevorzugte Ausführungsform des Verfahrens ist dadurch gekennzeichnet, dass die Wasserstoffrückgewinnungsanordnung eine Druckwechsel-Adsorptionsvorrichtung (PSA) zum Gewinnen des H-Recyclestroms aus dem Rückgewinnungsstrom aufweist. Auf diese Weise kann eine hohe Rückgewinnung an Wasserstoff im H-Recyclestrom erreicht werden. Ebenso sind die Druckverluste bei einer solchen Druckwechsel-Adsorptionsvorrichtung noch vertretbar. Eine hohe Wasserstoffreinheit ist zwar vorliegend grundsätzlich nicht erforderlich, kann aber erreicht werden. Es kann also sein, dass der H-Recyclestrom im Wesentlichen aus Wasserstoff besteht.

Die vorschlagsgemäße Anlage dient der Synthese von Methanol. Sie weist eine Synthesegasreaktoranordnung zum Gewinnen eines Synthesegasstroms mit Wasserstoff und Kohlenstoffoxiden aus einem kohlenstoffhaltigen Energieträgerstrom, einen Synthesegaskompressor zur Druckerhöhung des Synthesegasstroms, eine Methanol-Reaktoranordnung, welche eine erste Reaktorstufe aufweist, eine Wärmerückgewinnungsvorrichtung zum Rückgewinnen von Wärme aus dem Synthesegasstrom, eine Wasserstoffrückgewinnungsanordnung und einen Recyclekompressor auf.

Bei der vorschlagsgemäßen Anlage wird der druckerhöhte Synthesegasstrom zumindest teilweise der ersten Reaktorstufe zur teilweisen Umwandlung in Methanol zugeführt.

Weiter wird bei der vorschlagsgemäßen Anlage aus der Methanol-Reaktoranordnung ein Restgasstrom mit unreagierten Kohlenstoffoxiden gewonnen, welcher Restgasstrom dem Recyclekompressor zur Druckerhöhung des Restgasstroms zugeführt wird, wobei der druckerhöhte Restgasstrom der Methanol-Reaktoranordnung zur teilweisen Umwandlung in Methanol zugeführt wird, der Synthesegasstrom der Wärmerückgewinnungsvorrichtung und danach dem Synthesegaskompressor zugeführt wird, wobei der Wasserstoffrückgewinnungsvorrichtung ein Rückgewinnungsstrom aus einem unreagierten Restgas der ersten Reaktorstufe zum Gewinnen eines H-Recyclestroms zugeführt wird, welcher H-Recyclestrom unreagierten Wasserstoff aus dem unreagierten Restgas aufweist und welcher unreagierte Wasserstoff des H-Recyclestroms erneut der ersten Reaktorstufe zur zumindest teilweisen Umwandlung in Methanol zugeführt wird.

Die vorschlagsgemäße Anlage ist dadurch gekennzeichnet, dass der unreagierte Wasserstoff des H-Recyclestroms zumindest teilweise von der ersten Reaktorstufe bis zur erneuten Zuführung zur ersten Reaktorstufe durch den Recyclekompressor mit den unreagierten Kohlenstoffoxiden druckerhöht wird.

Merkmale, Vorteile und Eigenschaften der vorschlagsgemäßen Anlage entsprechen den Merkmalen, Vorteilen und Eigenschaften des vorschlagsgemäßen Verfahrens und umgekehrt.

Weitere Einzelheiten, Merkmale, Ziele und Vorteile der vorliegenden Erfindung werden nachfolgend anhand der nur Ausführungsbeispiele wiedergebenden Zeichnung erläutert. In der Zeichnung zeigt
- Fig. 1: schematisch das Fließbild einer Anlage zur Ausführung des vorschlagsgemäßen Verfahrens gemäß einem ersten Ausführungsbeispiel,
- Fig. 2: schematisch das Fließbild einer Anlage zur Ausführung des vorschlagsgemäßen Verfahrens gemäß einem zweiten Ausführungsbeispiel,
- Fig. 3: schematisch das Fließbild einer Anlage zur Ausführung des vorschlagsgemäßen Verfahrens gemäß einem dritten Ausführungsbeispiel,
- Fig. 4: schematisch das Fließbild einer Anlage zur Ausführung des vorschlagsgemäßen Verfahrens gemäß einem vierten Ausführungsbeispiel,
- Fig. 5: schematisch das Fließbild einer Anlage zur Ausführung des vorschlagsgemäßen Verfahrens gemäß einem fünften Ausführungsbeispiel,
- Fig. 6: schematisch das Fließbild einer Anlage zur Ausführung des vorschlagsgemäßen Verfahrens gemäß einem sechsten Ausführungsbeispiel,
- Fig. 7: schematisch das Fließbild einer Anlage zur Ausführung des vorschlagsgemäßen Verfahrens gemäß einem siebten Ausführungsbeispiel und
- Fig. 8: schematisch das Fließbild einer Anlage zur Ausführung des vorschlagsgemäßen Verfahrens gemäß einem achten Ausführungsbeispiel.

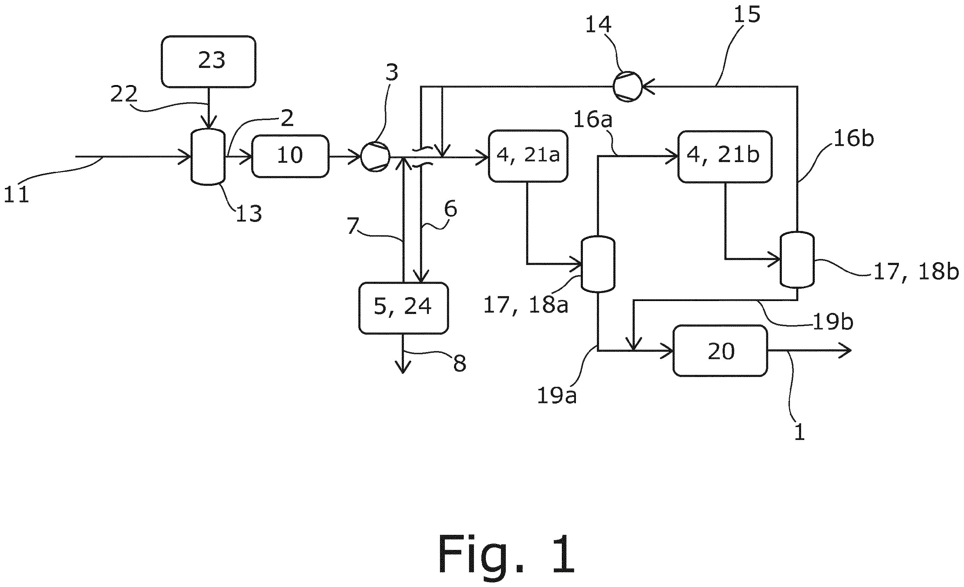

Die in der Fig. 1 gezeigte Anlage gemäß einem ersten Ausführungsbeispiel der vorschlagsgemäßen Anlage dient der Synthese von Methanol 1 und kann gemäß dem vorschlagsgemäßen Verfahren betrieben werden.

Ein im Wesentlichen aus Wasserstoff, Kohlenstoffmonoxid und Kohlenstoffdioxid bestehender Synthesegasstrom 2 wird aus einem durch Erdgas gebildeten und damit kohlenstoffhaltigen Energieträgerstrom 11 gewonnen, welcher einer Synthesegasreaktoranordnung 13 zugeführt wird. In der Synthesegasreaktoranordnung 13 findet eine autotherme Reformierung zum Gewinnen des Synthesegasstroms 2 statt. Für die autotherme Reformierung wird ein sauerstoffhaltiger Strom 22 zugeführt, welcher hier aus einer Luftzerlegungsvorrichtung 23 gewonnen wurde und im Wesentlichen aus Sauerstoff besteht. Die Luftzerlegungsvorrichtung 23 ist dabei zum Gewinnen eines Sauerstoffstroms - hier also des sauerstoffhaltigen Stroms 22 - aus der Umgebungsluft eingerichtet. Der Synthesegasstrom 2 wird mit einem Erzeugungsdruck von im Wesentlichen 60 bar gewonnen. Der Synthesegasstrom 2 wird zunächst einer Wärmerückgewinnungsanordnung 10 zugeführt, in welcher der Synthesegasstrom 2 abgekühlt wird und auf diese Weise ein Teil der bei der autothermen Reformierung erzeugten Wärme zurückgewonnen wird. Der Synthesegasstrom 2 wird danach einem Synthesegaskompressor 3 der Anlage zur weiteren Druckerhöhung zugeführt.

Anschließend wird der Synthesegasstrom 2 der ersten Reaktorstufe 21a einer Methanol-Reaktoranordnung 4 zugeführt, in welcher ersten Reaktorstufe 21a eine Methanolsynthese stattfindet und zumindest ein Teil des Synthesegasstroms 2 in Methanol 1 umgewandelt wird. Die Methanolsynthese findet bei einem Synthesedruck von über 60 bar statt, und zwar speziell bei einem Synthesedruck von im Wesentlichen 80 bar.

Die Anlage weist eine als Druckwechsel-Adsorptionsanlage 24 - welche auch als PSA bezeichnet werden kann - ausgebildete Wasserstoffrückgewinnungsanordnung 5 auf, welche aus einem Rückgewinnungsstrom 6 einen H-Recyclestrom 7 gewinnt, welcher H-Recyclestrom 7 im Wesentlichen aus Wasserstoff besteht. Ebenso wird das verbleibende Gas von der Wasserstoffrückgewinnungsanordnung 5 als Purgestrom 8 ausgegeben und anschließend in einer - hier nicht dargestellten - befeuerten Heizvorrichtung der Anlage verfeuert. Der H-Recyclestrom 7 wird dem Synthesegasstrom 2 zugeführt.

Wie in der Fig. 1 zu erkennen ist, weist die Anlage des ersten Ausführungsbeispiels ebenso einen Recyclekompressor 14 auf, welcher einen Restgasstrom 15 komprimiert. Der Restgasstrom 15 weist unreagiertes Restgas 16b auf, welches seinerseits im Wesentlichen diejenigen Bestandteile des Synthesegases aufweist, welche in der Methanol-Reaktoranordnung 4 nicht in Methanol 1 umgewandelt wurden. Entsprechend weist der Restgasstrom 15 insbesondere unreagierte Kohlenstoffoxide auf. Der somit druckerhöhte Restgasstrom 15 wird zu einem ersten Teil erneut der Methanol-Reaktoranordnung 4 zugeführt.

Das unreagierte Restgas 16a, b wird aus einer Methanol-Trennvorrichtung 17 der Methanol-Reaktoranordnung 4 gewonnen, welche hier zwei Kondensationsvorrichtungen 18a, b umfasst. Durch Kondensation wird in diesen jeweils das unreagierte Restgas 16a, b einerseits und ein jeweiliger Rohmethanolstrom 19a, b andererseits gewonnen. Die Rohmethanolströme 19a, b werden dann einer Destillation 20 der Anlage zugeführt, sodass das Methanol 1 aus den Rohmethanolströmen 19a, b gewonnen werden kann.

Bei der Anlage des Ausführungsbeispiels der Fig. 1 weist die Methanol-Reaktoranordnung 4 zwei prozesstechnisch hintereinander geschaltete Reaktorstufen 21a, b zur Methanolsynthese auf. Bei diesem Ausführungsbeispiel weist die erste Reaktorstufe 21a zwei zueinander parallel angeordnete isotherme Reaktoren und die zweite Reaktorstufe 21b einen einzelnen isothermen Reaktor auf. Jeder der beiden Kondensationsvorrichtungen 18a, b wird dabei der Produktstrom aus jeweils einer Reaktorstufe 21a, b zugeführt. Dabei wird diejenige Reaktorstufe 21a, welcher der Synthesegasstrom 2 direkt zugeführt wird, als erste Reaktorstufe 21a bezeichnet. Die Reaktorstufe 21b ist dieser dann in dem Sinne prozesstechnisch nachgelagert, dass ihr das unreagierte Restgas 16a aus der ersten Reaktorstufe 21a zur Umwandlung in Methanol 1 zugeführt wird.

Bei diesem Ausführungsbeispiel der Fig. 1 wird der Rückgewinnungsstrom 6 aus dem durch den Recyclekompressor druckerhöhten Restgasstrom 15 abgezweigt. Dieser dem Recyclekompressor 14 zugeführte Restgasstrom 15 wird nicht aus dem unreagierten Restgas 16a der ersten Reaktorstufe 21a gewonnen, sondern aus dem unreagierten Restgas 16b der der ersten Reaktorstufe 21a prozesstechnisch nachgelagerten und damit zweiten Reaktorstufe 21b.

Gleichwohl weist dieser Restgasstrom 15 neben den bereits genannten unreagierten Kohlenstoffoxiden auch unreagierten Wasserstoff aus der ersten Reaktorstufe 21a auf. Den jedweder unreagierte Wasserstoff aus dem Restgas 16a der ersten Reaktorstufe 21a wird der zweiten Reaktorstufe 21b zugeführt. Da auch in der zweiten Reaktorstufe 21b keine vollständige Reaktion des Wasserstoffs stattfindet, weist das unreagierte Restgas 16b der zweiten Reaktorstufe 21b auch unreagierten Wasserstoff aus der ersten Reaktorstufe 21a auf.

Da der Rückgewinnungsstrom 6 aus dem druckerhöhten Restgasstrom 15 abgezweigt wurde, weist auch der H-Recyclestrom 7 unreagierten Wasserstoff aus dem unreagierten Restgas 16a der ersten Reaktorstufe 21a auf. Speziell wird ein zweiter Teil des druckerhöhten Restgasstroms 15 als Rückgewinnungsstrom 6 abgezweigt. Dadurch, dass der H-Recyclestrom 7 dem druckerhöhten Synthesegasstrom 2 zugeführt wird, wird der unreagierte Wasserstoff aus dem Restgas 16a der ersten Reaktorstufe 21a im Rückgewinnungsstrom 6 - und damit auch aus dem H-Recyclestrom 7 - dieser ersten Reaktorstufe 21 wieder für die Umwandlung in Methanol zugeführt. Zwischen dem Verlassen der ersten Reaktorstufe 21a und der neuerlichen Zufuhr zu der ersten Reaktorstufe 21a hat der unreagierte Wasserstoff des H-Recyclestroms 7 jedoch als Bestandteil des Restgasstroms 15 eine Druckerhöhung durch den Recyclekompressor 14 erfahren, und zwar genau einmal und gemeinsam mit den unreagierten Kohlenstoffoxiden im Restgasstrom 15. Da der H-Recyclestrom 7 dem Synthesegasstrom 2 prozesstechnisch nach dem Synthesegaskompressor 3 zugeführt wird, findet also eine Druckerhöhung des Wasserstoffs im H-Recyclestrom 7 nicht statt. Der durch den Recyclekompressor 14 komprimierte Restgasstrom 15 wird dann wiederum zu dem bereits erwähnten ersten Teil direkt der ersten Reaktorstufe 21a zugeführt.

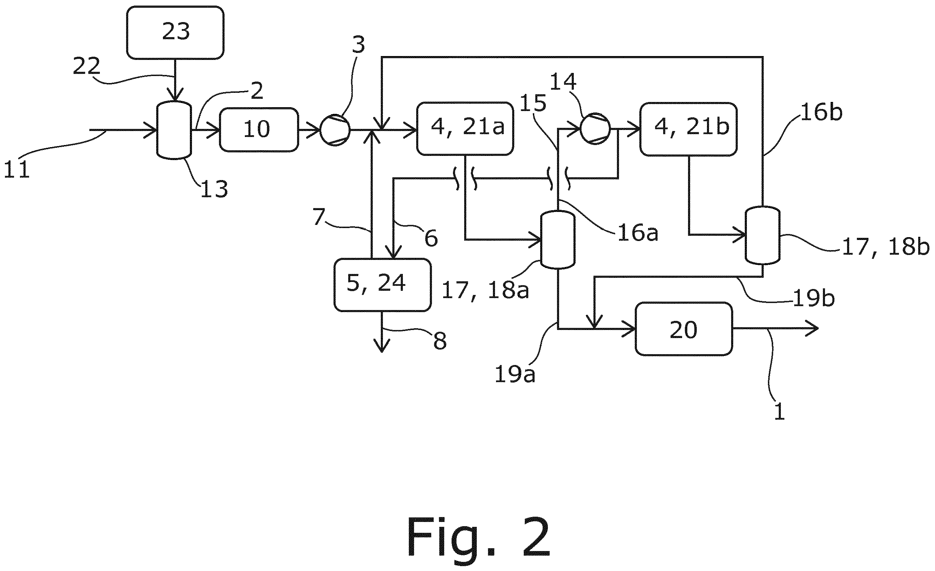

Das zweite Ausführungsbeispiel der vorschlagsgemäßen Anlage, dargestellt in der Fig. 2, unterscheidet sich von dem Ausführungsbeispiel der Fig. 1 darin, dass der Recyclekompressor 14 prozesstechnisch zwischen der ersten Reaktorstufe 21a und der ihr nachgelagerten Reaktorstufe 21b angeordnet ist. Folglich wird der dem Recyclekompressor 14 zugeführte Restgasstrom 15 aus dem unreagierten Restgas 16a der ersten Reaktorstufe 21a gewonnen. Der durch den Recyclekompressor 14 komprimierte Restgasstrom 15 mit den unreagierten Kohlenstoffoxiden wird der der ersten Reaktorstufe 21a nachgelagerten Reaktorstufe 21b zugeführt. Das unreagierte Restgas 16b aus dieser Reaktorstufe 21b wird ohne weitere Komprimierung zurück zur ersten Reaktorstufe 21a geführt. Der Rückgewinnungsstrom 6 wird - anders als bei dem ersten Ausführungsbeispiel - aus dem unreagierten Restgas 16a der ersten Reaktorstufe 21a gewonnen, wobei ebenfalls in Übereinstimmung mit dem ersten Ausführungsbeispiel das Abzweigen des Rückgewinnungsstroms 6 dem Recyclekompressor 14 prozesstechnisch nachgelagert erfolgt. Folglich findet auch bei dem zweiten Ausführungsbeispiel eine Druckerhöhung des unreagierten Wasserstoffs aus dem Restgas 16a der ersten Reaktorstufe 21a im H-Recyclestrom 7 mit den unreagierten Kohlenstoffoxiden durch den Recyclekompressor 14 genau einmal statt, bevor dieser unreagierte Wasserstoff wieder der ersten Reaktorstufe 21a zugeführt wird.

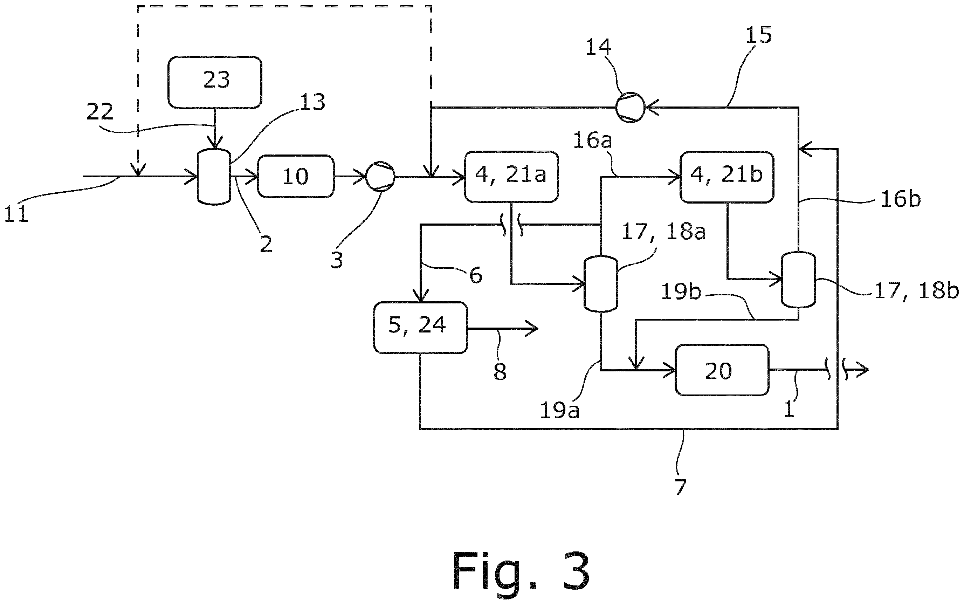

Bei dem dritten Ausführungsbeispiel der Fig. 3 wird der Rückgewinnungsstrom 6 ähnlich wie bei dem zweiten Ausführungsbeispiel aus dem Restgas 16a der ersten Reaktorstufe 21a gewonnen. Im Gegensatz zum zweiten Ausführungsbeispiel ist jedoch kein Recyclekompressor 14 zwischen der ersten Reaktorstufe 21a und der zweiten Reaktorstufe 21b angeordnet. Vielmehr ist der Recyclekompressor 14 wie bei dem ersten Ausführungsbeispiel der zweiten Reaktorstufe 21b prozesstechnisch nachgelagert angeordnet.

Anders als sowohl dem ersten Ausführungsbeispiel und dem zweiten Ausführungsbeispiel wird bei dem dritten Ausführungsbeispiel der H-Recyclestrom 7 dem Restgas 16b der der ersten Reaktorstufe 21a nachgelagerten zweiten Reaktorstufe 21b zugeführt. Speziell erfolgt diese Zuführung vor der Druckerhöhung durch den Recyclekompressor 14. Der Wasserstoff in dem H-Recyclestrom 7 entsprechend dem unreagierten Wasserstoff aus dem Restgas 16a der ersten Reaktorstufe 21a im Rückgewinnungsstrom 6 erhält auf diese Weise mit dem sonstigen unreagierten Restgas 16b der zweiten Reaktorstufe 21b und insbesondere mit unreagierten Kohlenstoffoxiden eine Druckerhöhung durch den Recyclekompressor 14. Diese Druckerhöhung erfolgt vor der erneuten Zuführung dieses unreagierten Wasserstoffes zu der ersten Reaktorstufe 21a, was die mangels Synthesegaskompressors fehlende Druckerhöhung kompensiert.

Zusätzlich ist bei dem dritten Ausführungsbeispiel vorgesehen, dass ein Teil des druckerhöhten Restgasstroms 15 abgezweigt und dem Energieträgerstrom 11 zugeführt wird. Dieser abgezweigte Teil des druckerhöhten Restgasstroms 15 erfährt eine weitere Druckerhöhung durch den Synthesegaskompressor 2. Für den nicht abgezweigten Teil des Restgasstroms 15 fand genau einmal eine Druckerhöhung durch den Recyclekompressor 14 statt. Es ist aber auch möglich, auf dieses Abzweigen eines Teils des druckerhöhten Restgasstroms 15 zu verzichten.

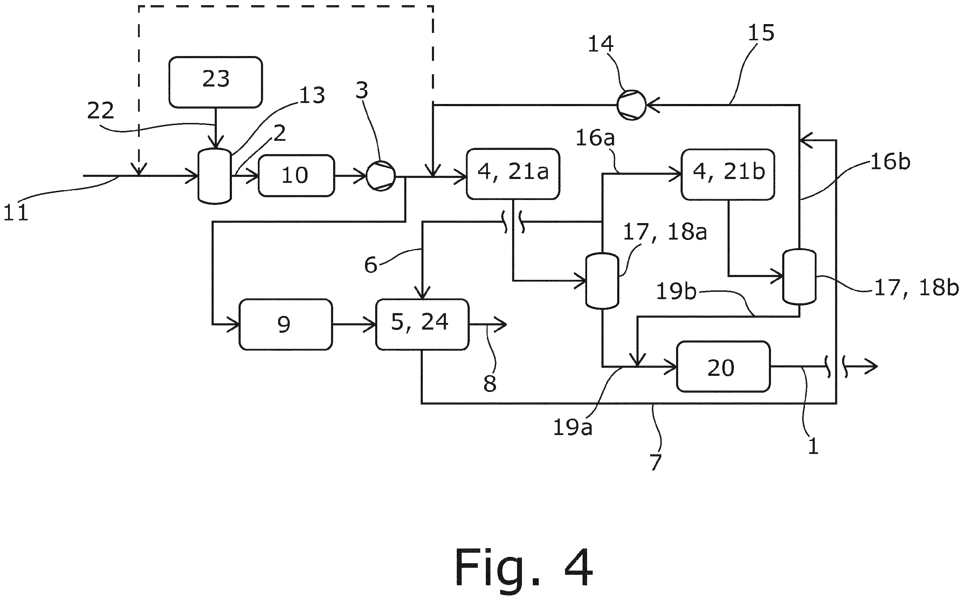

Die Anlage gemäß dem vierten Ausführungsbeispiel der Fig. 4 entspricht dem dritten Ausführungsbeispiel der Fig. 3. Sie weist jedoch eine Wassergas-Shift-Reaktionsvorrichtung 9 auf, welcher ein Teil des druckerhöhten Synthesegasstroms 2 zugeführt wird. Die in der Wassergas-Shift-Reaktionsvorrichtung 9 ablaufende Wassergas-Shift-Reaktion führt zu einer Erhöhung des Wasserstoffanteils in dem abgezweigten Teil des druckerhöhten Synthesegasstroms 2. Der auf diese Weise abgezweigte und der Wassergas-Shift-Reaktion unterzogene Teil des Synthesegasstrom 2 aus der Wassergas-Shift-Reaktionsvorrichtung 9 bildet hier einen weiteren Rückgewinnungsstrom, welcher zusammen mit der Rückgewinnungsstrom 6 der Wasserstoffrückgewinnungsanordnung 5 zugeführt wird. Ebenso wie bei dem Ausführungsbeispiel der Fig. 3 wird der H-Recyclestrom 7 dem Restgas 16b der der ersten Reaktorstufe 21a nachgelagerten zweiten Reaktorstufe 21b zugeführt, sodass also auch bei diesem Ausführungsbeispiel eine Druckerhöhung durch den Recyclekompressor 14 mit den unreagierten Kohlenstoffoxiden erfolgt.

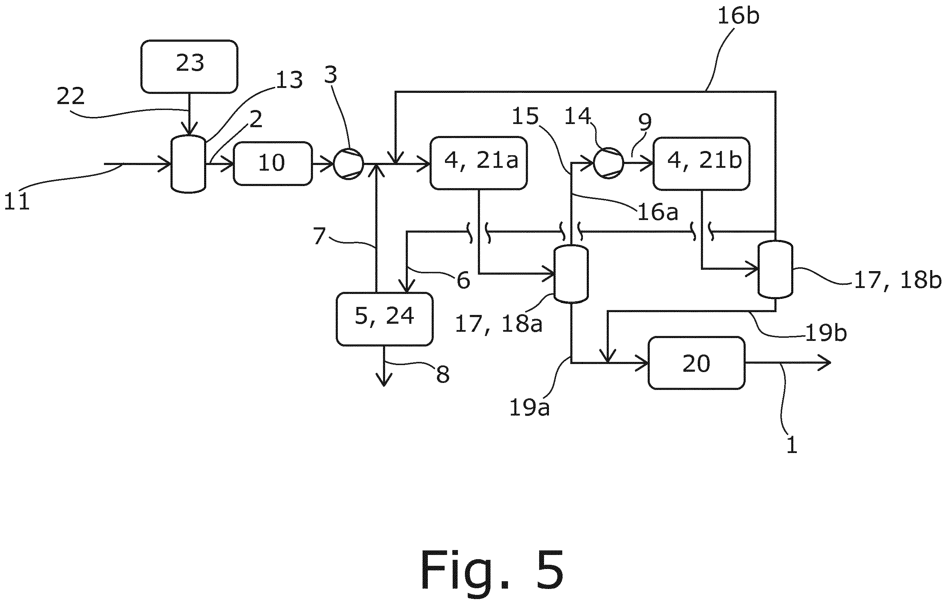

Das fünfte Ausführungsbeispiel der Fig. 5 sieht eine Anordnung des Recyclekompressors 14 zwischen den Reaktorstufen 21a, b der Methanol-Reaktoranordnung 4 vor wie bei dem zweiten Ausführungsbeispiel, auf welchem das fünfte Ausführungsbeispiel auch basiert. Im Unterschied zum zweiten Ausführungsbeispiel wird der Rückgewinnungsstrom 6 aus dem Restgas 16b der zweiten Reaktorstufe 21b gewonnen. Der Wasserstoff in diesem Rückgewinnungsstrom 6 und damit auch im H-Recyclestrom 7 hat dabei eine Druckerhöhung durch den Recyclekompressor 14 erfahren, und zwar speziell noch vor Zuführung zu der zweiten Reaktorstufe 21b.

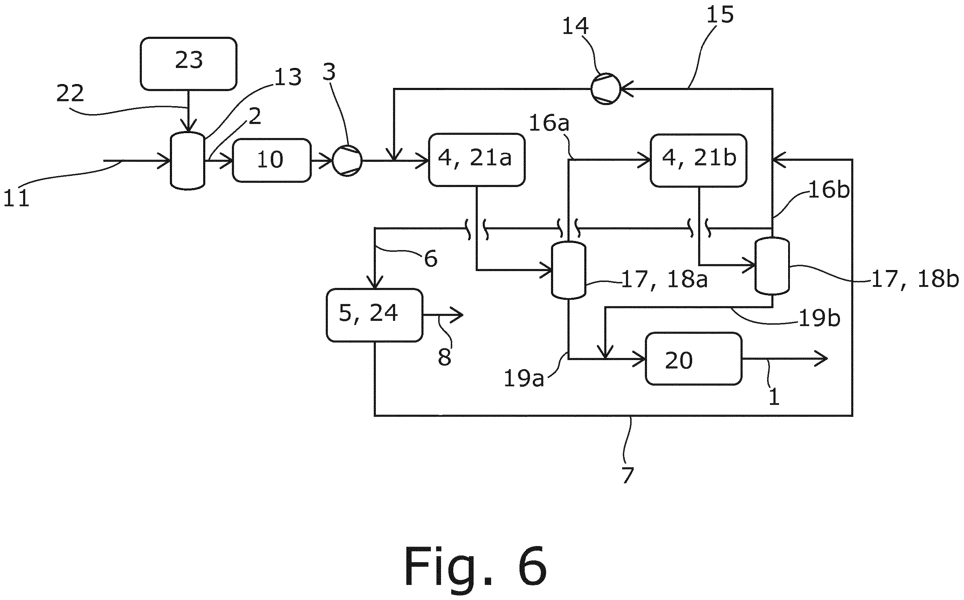

Das sechste Ausführungsbeispiel der Fig. 6 basiert grundsätzlich auf dem ersten Ausführungsbeispiel der Fig. 1. Abweichend davon und insoweit in Übereinstimmung mit dem fünften Ausführungsbeispiel der Fig. 5 wird der Rückgewinnungsstrom 6 aus dem unreagierten Restgas 16b der zweiten Reaktorstufe 21b gewonnen. Ebenfalls abweichend von dem ersten Ausführungsbeispiel der Fig. 1 und insoweit in Übereinstimmung mit dem dritten und vierten Ausführungsbeispiel der Fig, 3 und 4 wird der H-Recyclestrom 7 wieder dem Restgas 16b der der ersten Reaktorstufe 21a nachgelagerten zweiten Reaktorstufe 21b zugeführt. Diese Zuführung erfolgt prozesstechnisch nachgelagert zur Abzweigung des Rückgewinnungsstroms 6.

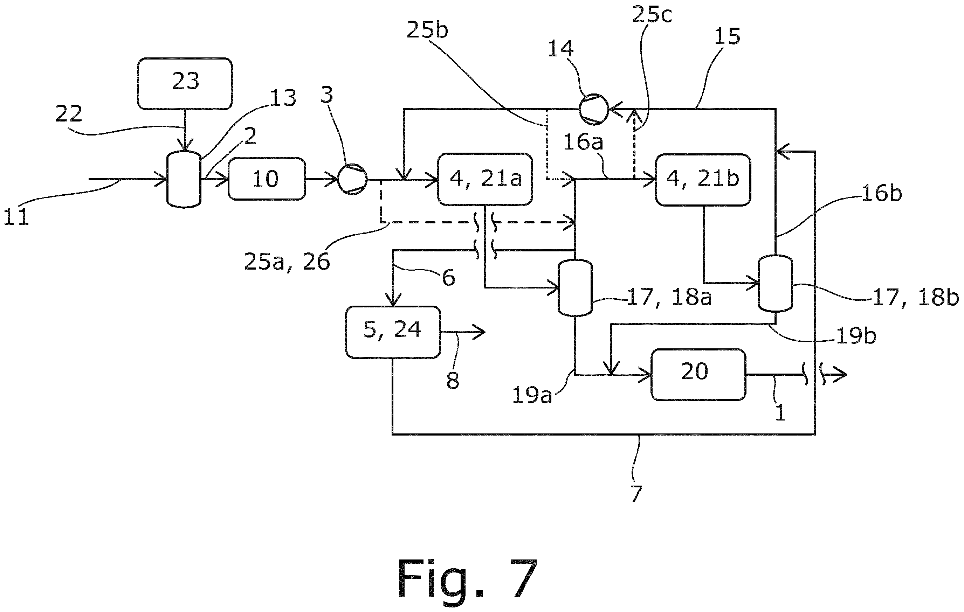

Das siebte Ausführungsbeispiel der Fig. 7 basiert zunächst auf dem dritten Ausführungsbeispiel der Fig. 3, allerdings ohne die Abzweigung eines Teils des druckerhöhten Restgasstroms 15 zum Energieträgerstrom 11. Das siebte Ausführungsbeispiel zeigt drei jeweils alternative Verschaltungsvarianten des dritten Ausführungsbeispiels. Insoweit umfasst das siebte Ausführungsbeispiel drei Unterausführungsbeispiele.

Die erste Variante sieht einen ersten Umgehungsstrom 25a vor, welcher von dem druckerhöhten Synthesegasstrom 2 abgezweigt und dem unreagierten Restgas 16a der ersten Reaktorstufe 21a zugeführt wird. Die Zuführung zum unreagierten Restgas 16a der ersten Reaktorstufe 21a erfolgt prozesstechnisch nachgelagert zur Abzweigung des Rückgewinnungsstroms 6. Auf diese Weise wird also ein Teil des Synthesegasstroms 2 zum Bilden eines weiteren Synthesegasstroms 26 abgezweigt, welcher dem ersten Umgehungsstrom 25a entspricht und welcher die erste Reaktorstufe 21a umgeht. Gemäß der Darstellung der Fig. 7 erfolgt diese Abzweigung prozesstechnisch vor der Zuführung des druckerhöhten Restgasstroms 15. Denkbar wäre aber auch, dass diese Abzweigung prozesstechnisch nach der Zuführung des druckerhöhten Restgasstroms 15 erfolgt.

Die zweite, zu der ersten alternative Variante sieht einen zweiten Umgehungsstrom 25b vor, welcher aus dem druckerhöhten Restgasstrom 15 abgezweigt und dem unreagierten Restgas 16a der ersten Reaktorstufe 21a zugeführt wird, und zwar wiederum der Abzweigung des Rückgewinnungsstroms 6 prozesstechnisch nachgelagert. Auf diese Weise erfolgt also ebenfalls eine teilweise Umgehung der ersten Reaktorstufe 21a durch den druckerhöhten Restgasstrom 15.

Die dritte, zu den ersten beiden Varianten alternative Variante sieht einen dritten Umgehungsstrom 25c vor, welcher der Abzweigung des Rückgewinnungsstroms 6 prozesstechnisch nachgelagert aus dem unreagierten Restgas 16a der ersten Reaktorstufe 21a abgezweigt und dem Restgasstrom 15 vor der Druckerhöhung zugeführt wird. Auf diese Weise umgeht also ein Teil des unreagierten Restgases 16a der ersten Reaktorstufe 21a zunächst die zweite Reaktorstufe 21b.

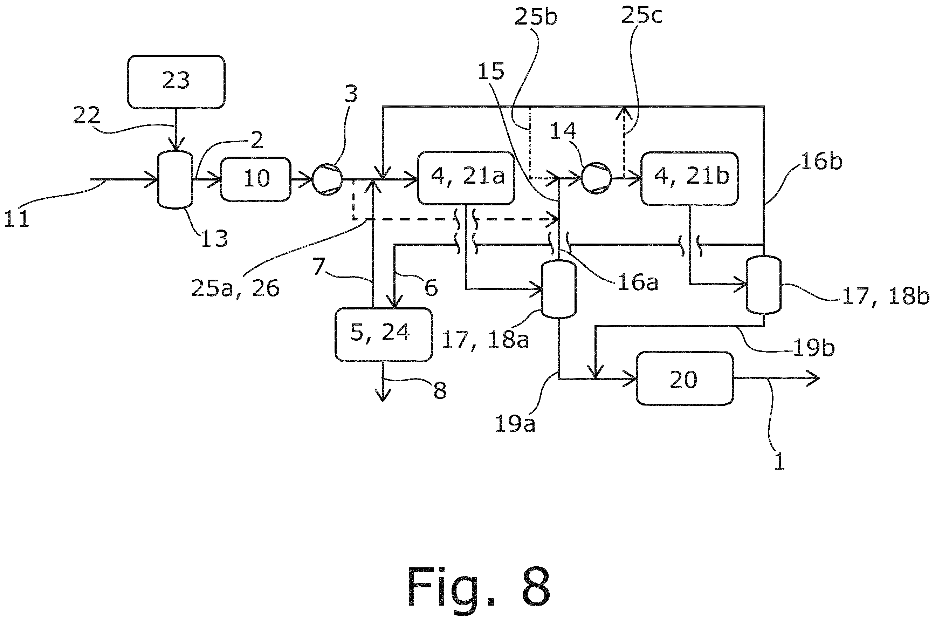

Das achte Ausführungsbeispiel der Fig. 8 basiert auf dem fünften Ausführungsbeispiel der Fig. 5 und zeigt ebenfalls drei jeweils alternative Verschaltungsvarianten, hier bezogen auf das fünfte Ausführungsbeispiel. Diese drei Verschaltungsvarianten entsprechenden Verschaltungsvarianten des siebten Ausführungsbeispiels der Fig. 7 mit den Umgehungsströmen 25a, 25b und 25c.

## Patentansprüche

1. Verfahren zur Synthese von Methanol (1), wobei ein kohlenstoffhaltiger Energieträgerstrom (11) einer Synthesegasreaktoranordnung (13) zum Gewinnen eines Synthesegasstroms (2) mit Wasserstoff und Kohlenstoffoxiden zugeführt wird, wobei der Synthesegasstrom (2) einer Wärmerückgewinnungsvorrichtung (10) zum Rückgewinnen von Wärme aus dem Synthesegasstrom (2) und danach einem Synthesegaskompressor (3) zur Druckerhöhung zugeführt wird, wobei der druckerhöhte Synthesegasstrom (2) zumindest teilweise einer ersten Reaktorstufe (21a) einer Methanol-Reaktoranordnung (4) zur teilweisen Umwandlung in Methanol (1) zugeführt wird, wobei aus der Methanol-Reaktoranordnung (4) ein Restgasstrom (15) mit unreagierten Kohlenstoffoxiden gewonnen wird, welcher Restgasstrom (15) einem Recyclekompressor (14) zur Druckerhöhung des Restgasstroms (15) zugeführt wird, wobei der druckerhöhte Restgasstrom (15) der Methanol-Reaktoranordnung (4) zur teilweisen Umwandlung in Methanol (1) zugeführt wird , wobei ein Rückgewinnungsstrom (6) aus einem unreagierten Restgas (16a) der ersten Reaktorstufe (21a) einer Wasserstoffrückgewinnungsanordnung (5) zum Gewinnen eines H-Recyclestroms (7) zugeführt wird, welcher H-Recyclestrom (7) unreagierten Wasserstoff aus dem unreagierten Restgas (16a) aufweist, welcher unreagierte Wasserstoff des H-Recyclestroms (7) erneut der ersten Reaktorstufe (21a) zur zumindest teilweisen Umwandlung in Methanol (1) zugeführt wird, **dadurch gekennzeichnet, dass** der unreagierte Wasserstoff des H-Recyclestroms (7) zumindest teilweise von der ersten Reaktorstufe (21a) bis zur erneuten Zuführung zur ersten Reaktorstufe (21a) durch den Recyclekompressor (14) mit den unreagierten Kohlenstoffoxiden druckerhöht wird.

2. Verfahren nach Anspruch 1, **dadurch gekennzeichnet, dass** die Methanol-Reaktoranordnung (4) eine Methanol-Trennvorrichtung (17) zum Gewinnen des unreagierten Restgases (16a) der ersten Reaktorstufe (21a) und eines Rohmethanolstroms (19a) der ersten Reaktorstufe (21a) umfasst, insbesondere, dass die Methanol-Trennvorrichtung (17) eine Kondensationsvorrichtung (18a) zum Gewinnen des unreagierten Restgases (16a) der ersten Reaktorstufe (21a) und des Rohmethanolstroms (19a) der ersten Reaktorstufe (21a) durch Kondensation umfasst.

3. Verfahren nach Anspruch 1 oder 2, **dadurch gekennzeichnet, dass** ein Teil des druckerhöhten Restgasstroms (15) abgezweigt und der Synthesegasreaktoranordnung (13) zugeführt wird, vorzugsweise dass der abgezweigte Teil des druckerhöhten Restgasstroms (15) dem Energieträgerstrom (11) zugeführt wird.

4. Verfahren nach einem der Ansprüche 1 bis 3, **dadurch gekennzeichnet, dass** die Methanol-Reaktoranordnung (4) eine Vielzahl von prozesstechnisch hintereinander geschalteten Reaktorstufen (21a, b) zur Methanolsynthese aufweist, vorzugsweise, dass der Recyclekompressor (14) prozesstechnisch zwischen zwei Reaktorstufen (21a, b) angeordnet ist, insbesondere, dass durch die Methanol-Trennvorrichtung (17) ein jeweiliges unreagiertes Restgas (16a, b) aus jeder der Vielzahl von Reaktorstufen (21a, b) gewonnen wird.

5. Verfahren nach Anspruch 4, **dadurch gekennzeichnet, dass** der H-Recyclestrom (7) dem unreagierten Restgas (16b) einer prozesstechnisch der ersten Reaktorstufe (21a) nachgelagerten Reaktorstufe (21b) zugeführt wird, vorzugsweise, dass der H-Recyclestrom (7) dem Recyclekompressor (14) zur Druckerhöhung gemeinsam mit dem Restgasstrom (15) zugeführt wird.

6. Verfahren nach Anspruch 4 oder 5, **dadurch gekennzeichnet, dass** der Restgasstrom (15) aus einer prozesstechnisch der ersten Reaktorstufe (21a) nachgelagerten Reaktorstufe (21b) gewonnen wird, insbesondere, dass der Recyclekompressor (14) den druckerhöhten Restgasstrom (15) der ersten Reaktorstufe (21a) zuführt.

7. Verfahren nach Anspruch 6, **dadurch gekennzeichnet, dass** der Restgasstrom (15) aus einer prozesstechnisch zuletztgelagerten Reaktorstufe (21b) der Vielzahl von Reaktorstufen (21a, b) gewonnen wird.

8. Verfahren nach einem der Ansprüche 1 bis 7, **dadurch gekennzeichnet, dass** der Rückgewinnungsstrom (6) zumindest teilweise aus dem unreagierten Restgas (16a) der ersten Reaktorstufe (21a) abgezweigt wird, weiter vorzugsweise, dass der Rückgewinnungsstrom (6) zumindest teilweise dem Recyclekompressor (14) prozesstechnisch vorgelagert abgezweigt wird.

9. Verfahren nach Anspruch 8, **dadurch gekennzeichnet, dass** der Rückgewinnungsstrom (6) der Wasserstoffrückgewinnungsanordnung (5) mit einem Zuführungsdruck zugeführt wird, welcher höher ist als ein Restgasdruck, mit welchem der Restgasstrom (15) aus der Methanol-Reaktoranordnung gewonnen wird, vorzugsweise, dass der Rückgewinnungsstrom (6) zumindest teilweise dem Recyclekompressor (14) prozesstechnisch nachgelagert aus dem Restgasstrom (15) abgezweigt wird.

10. Verfahren nach einem der Ansprüche 1 bis 9, **dadurch gekennzeichnet, dass** der H-Recyclestrom (7) dem insbesondere druckerhöhten Synthesegasstrom (2) zugeführt wird.

11. Verfahren nach einem der Ansprüche 1 bis 10, **dadurch gekennzeichnet, dass** zum Gewinnen des Synthesegasstroms (2) ein sauerstoffhaltiger Strom (22) der Synthesegasreaktoranordnung (13) zugeführt wird, insbesondere, dass in der Synthesegasreaktoranordnung (13) der Synthesegasstrom (2) durch eine autotherme Reformierung oder eine partielle Oxidation aus dem kohlenstoffhaltigen Energieträgerstrom (11) gewonnen wird, weiter vorzugsweise, dass der sauerstoffhaltige Strom (22) aus einer Luftzerlegungsvorrichtung (23) zum Gewinnen eines Sauerstoffstroms aus einer Umgebungsluft gewonnen wird, weiter, insbesondere, dass der sauerstoffhaltige Strom (22) im Wesentlichen aus Sauerstoff besteht.

12. Verfahren nach einem der Ansprüche 1 bis 11, **dadurch gekennzeichnet, dass** der H-Recyclestrom (7) dem Energieträgerstrom (11), insbesondere der Synthesegasreaktoranordnung (13) prozesstechnisch vorgelagert, zugeführt wird, vorzugsweise, dass die Wasserstoffrückgewinnungsanordnung (5) einen Purgestrom (8) ausgibt, welcher weiter insbesondere zur Verfeuerung abgeführt wird.

13. Verfahren nach einem der Ansprüche 1 bis 12, **dadurch gekennzeichnet, dass** der H-Recyclestrom (7) einen höheren molaren Anteil an Wasserstoff als der Rückgewinnungsstrom (6) aufweist, vorzugsweise, dass der H-Recyclestrom (7) einen höheren molaren Anteil an Wasserstoff als der Purgestrom (8) aufweist.

14. Verfahren nach einem der Ansprüche 1 bis 13, **dadurch gekennzeichnet, dass** die Wasserstoffrückgewinnungsanordnung (5) eine Druckwechsel-Adsorptionsvorrichtung (24) zum Gewinnen des H-Recyclestroms (7) aus dem Rückgewinnungsstrom (6) aufweist, weiter vorzugsweise, dass der H-Recyclestrom (7) im Wesentlichen aus Wasserstoff besteht.

15. Verfahren nach einem der Ansprüche 1 bis 14, **dadurch gekennzeichnet, dass** der unreagierte Wasserstoff des Rückgewinnungsstroms (6) von der ersten Reaktorstufe (21a) bis zur erneuten Zuführung zur ersten Reaktorstufe (21a) durch den Recyclekompressor (14) mit den unreagierten Kohlenstoffoxiden genau einmal druckerhöht wird.

16. Anlage zur Synthese von Methanol (1) mit einer Synthesegasreaktoranordnung (13) zum Gewinnen eines Synthesegasstroms (2) mit Wasserstoff und Kohlenstoffoxiden aus einem kohlenstoffhaltigen Energieträgerstrom (11), mit einem Synthesegaskompressor (3) zur Druckerhöhung des Synthesegasstroms (2), mit einer Methanol-Reaktoranordnung (4), welche eine erste Reaktorstufe (21a) aufweist, mit einer Wärmerückgewinnungsvorrichtung (10) zum Rückgewinnen von Wärme aus dem Synthesegasstrom (2), mit einer Wasserstoffrückgewinnungsanordnung (5) und mit einem Recyclekompressor (14), wobei der druckerhöhte Synthesegasstrom (2) zumindest teilweise der ersten Reaktorstufe (21a) zur teilweisen Umwandlung in Methanol (1) zugeführt wird, wobei aus der Methanol-Reaktoranordnung (4) ein Restgasstrom (15) mit unreagierten Kohlenstoffoxiden gewonnen wird, welcher Restgasstrom (15) dem Recyclekompressor (14) zur Druckerhöhung des Restgasstroms (15) zugeführt wird, wobei der druckerhöhte Restgasstrom (15) der Methanol-Reaktoranordnung (4) zur teilweisen Umwandlung in Methanol (1) zugeführt wird, wobei der Synthesegasstrom (2) der Wärmerückgewinnungsvorrichtung (10) und danach dem Synthesegaskompressor (3) zugeführt wird, wobei der Wasserstoffrückgewinnungsvorrichtung (5) ein Rückgewinnungsstrom (6) aus einem unreagierten Restgas (16a) der ersten Reaktorstufe (21a) zum Gewinnen eines H-Recyclestroms (7) zugeführt wird, welcher H-Recyclestrom (7) unreagierten Wasserstoff aus dem unreagierten Restgas (16a) aufweist, weleher unreagierte Wasserstoff des H-Recyclestroms (7) erneut der ersten Reaktorstufe (21a) zur zumindest teilweisen Umwandlung in Methanol (1) zugeführt wird, **dadurch gekennzeichnet, dass** der unreagierte Wasserstoff des H-Recyclestroms (7) zumindest teilweise von der ersten Reaktorstufe (21a) bis zur erneuten Zuführung zur ersten Reaktorstufe (21a) durch den Recyclekompressor (14) mit den unreagierten Kohlenstoffoxiden druckerhöht wird.

## Claims

1. A method for the synthesis of methanol (1), wherein a carbon-containing energy source stream (11) is fed to a synthesis gas reactor unit (13) in order to obtain a synthesis gas stream (2) with hydrogen and oxides of carbon, wherein the synthesis gas stream (2) is fed to a heat recovery device (10) in order to recover heat from the synthesis gas stream (2) and thereafter to a synthesis gas compressor (3) in order to increase the pressure, wherein at least a portion of the pressurized synthesis gas stream (2) is fed to a first reactor stage (21a) of a methanol reactor unit (4) for partial conversion into methanol (1), wherein a residual gas stream (15) with unreacted oxides of carbon is obtained from the methanol reactor unit (4), the residual gas stream (15) being fed to a recycle compressor (14) in order to increase the pressure of the residual gas stream (15), wherein the pressurized residual gas stream (15) is fed to the methanol reactor unit (4) for partial conversion into methanol (1), wherein a recovery stream (6) formed by an unreacted residual gas (16a) from the first reactor stage (21a) is fed to a hydrogen recovery unit (5) in order to obtain a H recycle stream (7), the H recycle stream (7) having unreacted hydrogen from the unreacted residual gas (16a), the unreacted hydrogen of the H recycle stream (7) being fed once again to the first reactor stage (21a) for at least partial conversion into methanol (1), **characterized in that** the pressure of at least a portion of the unreacted hydrogen of the H recycle stream (7) from the first reactor stage (21a) up to its feed once again into the first reactor stage (21a) is increased along with the unreacted oxides of carbon by means of the recycle compressor (14).

2. The method as claimed in claim 1, **characterized in that** the methanol reactor unit (4) comprises a methanol separation device (17) for obtaining the unreacted residual gas (16a) from the first reactor stage (21a) and a raw methanol stream (19a) from the first reactor stage (21a), in particular **in that** the methanol separation device (17) comprises a condensation device (18a) for obtaining the unreacted residual gas (16a) from the first reactor stage (21a) and the raw methanol stream (19a) from the first reactor stage (21a) by condensation.

3. The method as claimed in claim 1 or claim 2, **characterized in that** a portion of the pressurized residual gas stream (15) is diverted and fed to the synthesis gas reactor unit (13), preferably **in that** the diverted portion of the pressurized residual gas stream (15) is fed to the energy source stream (11).

4. The method as claimed in one of claims 1 to 3, **characterized in that** the methanol reactor unit (4) has a plurality of reactor stages (21a, b) for the synthesis of methanol which are operationally connected in series, preferably **in that** the recycle compressor (14) is operationally disposed between two reactor stages (21a, b), in particular **in that** by means of the methanol separation device (17), a respective unreacted residual gas (16a, b) is obtained from each of the plurality of reactor stages (21a, b).

5. The method as claimed in claim 4, **characterized in that** the H recycle stream (7) is fed to the unreacted residual gas (16b) from a reactor stage (21b) which is operationally downstream of the first reactor stage (21a), preferably **in that** the H recycle stream (7) is fed to the recycle compressor (14) together with the residual gas stream (15) in order to increase the pressure.

6. The method as claimed in claim 4 or claim 5, **characterized in that** the residual gas stream (15) is obtained from a reactor stage (21b) which is operationally downstream of the first reactor stage (21a), in particular **in that** the recycle compressor (14) feeds the pressurized residual gas stream (15) to the first reactor stage (21a).

7. The method as claimed in claim 6, **characterized in that** the residual gas stream (15) is obtained from a reactor stage (21b) of the plurality of reactor stages (21a, b) which is in the last operational position.

8. The method as claimed in one of claims 1 to 7, **characterized in that** at least a portion of the recovery stream (6) is diverted from the unreacted residual gas (16a) of the first reactor stage (21a), more preferably **in that** at least a portion of the recovery stream (6) is diverted operationally upstream of the recycle compressor (14).

9. The method as claimed in claim 8, **characterized in that** the recovery stream (6) is fed to the hydrogen recovery unit (5) at a feed pressure which is higher than a residual gas pressure at which the residual gas stream (15) is obtained from the methanol reactor unit, preferably **in that** at least a portion of the recovery stream (6) is diverted from the residual gas stream (15) operationally downstream of the recycle compressor (14).

10. The method as claimed in one of claims 1 to 9, **characterized in that** the H recycle stream (7) is fed to the synthesis gas stream (2) which in particular has been pressurized.

11. The method as claimed in one of claims 1 to 10, **characterized in that** in order to obtain the synthesis gas stream (2), an oxygen-containing stream (22) is fed to the synthesis gas reactor unit (13), in particular in that in the synthesis gas reactor unit (13), the synthesis gas stream (2) is obtained by means of autothermal reforming or a partial oxidation of the carbon-containing energy source stream (11), more preferably **in that** the oxygen-containing stream (22) is obtained from an air separation device (23) in order to obtain a stream of oxygen from ambient air, more particularly **in that** the oxygen-containing stream (22) substantially consists of oxygen.

12. The method as claimed in one of claims 1 to 11, **characterized in that** the H recycle stream (7) is fed to the energy source stream (11), in particular operationally upstream of the synthesis gas reactor unit (13), preferably **in that** the hydrogen recovery unit (5) outputs a purge (8) which more particularly is discharged for combustion.

13. The method as claimed in one of claims 1 to 12, **characterized in that** the H recycle stream (7) has a higher molar proportion of hydrogen than the recovery stream (6), preferably **in that** the H recycle stream (7) has a higher molar proportion of hydrogen than the purge (8).

14. The method as claimed in one of claims 1 to 13, **characterized in that** the hydrogen recovery unit (5) has a pressure swing adsorption device (24) for obtaining the H recycle stream (7) from the recovery stream (6), more preferably **in that** the H recycle stream (7) substantially consists of hydrogen.

15. The method as claimed in one of claims 1 to 14, **characterized in that** the pressure of the unreacted hydrogen of the recovery stream (6) from the first reactor stage (21a) up to its feed once again into the first reactor stage (21a) is increased exactly once along with the unreacted oxides of carbon by means of the recycle compressor (14).

16. A unit for the synthesis of methanol (1), with a synthesis gas reactor unit (13) for obtaining a synthesis gas stream (2) with hydrogen and oxides of carbon from a carbon-containing energy source stream (11), with a synthesis gas compressor (3) for pressurizing the synthesis gas stream (2), with a methanol reactor unit (4) which has a first reactor stage (21a), with a heat recovery device (10) for recovering heat from the synthesis gas stream (2), with a hydrogen recovery unit (5) and with a recycle compressor (14), wherein at least a portion of the pressurized synthesis gas stream (2) is fed to the first reactor stage (21a) for partial conversion into methanol (1), wherein a residual gas stream (15) with unreacted oxides of carbon is obtained from the methanol reactor unit (4), the residual gas stream (15) being fed to the recycle compressor (14) in order to increase the pressure of the residual gas stream (15), wherein the pressurized residual gas stream (15) is fed to the methanol reactor unit (4) for partial conversion into methanol (1), wherein the synthesis gas stream (2) is fed to the heat recovery device (10) and thereafter to the synthesis gas compressor (3), wherein a recovery stream (6) formed by an unreacted residual gas (16a) from the first reactor stage (21a) is fed to the hydrogen recovery unit (5) in order to obtain a H recycle stream (7), the H recycle stream (7) having unreacted hydrogen from the unreacted residual gas (16a), the unreacted hydrogen of the H recycle stream (7) being fed once again to the first reactor stage (21a) for at least partial conversion into methanol (1), **characterized in that** the pressure of at least a portion of the unreacted hydrogen of the H recycle stream (7) from the first reactor stage (21a) up to its feed once again into the first reactor stage (21a) is increased along with the unreacted oxides of carbon by means of the recycle compressor (14).

## Revendications

1. Procédé de synthèse de méthanol (1), consistant à introduire un flux de vecteur d'énergie (11) carboné dans un système de réacteur de gaz de synthèse (13) afin d'obtenir un flux de gaz de synthèse (2) comportant de l'hydrogène et des oxydes de carbone, le flux de gaz de synthèse (2) étant introduit dans un dispositif de récupération de chaleur (10) afin de récupérer de la chaleur à partir du flux de gaz de synthèse (2) puis dans un compresseur de gaz de synthèse (3) afin d'y subir une augmentation de la pression, le flux de gaz de synthèse (2) sous pression augmentée étant introduit, au moins partiellement, dans un premier étage de réacteur (21a) d'un système de réacteur de méthanol (4) afin d'y être partiellement transformé en méthanol (1), le système de réacteur de méthanol (4) produisant un flux gazeux résiduel (15) comportant des oxydes de carbone n'ayant pas réagi, le flux gazeux résiduel (15) étant introduit dans un compresseur de recyclage (14) afin que le flux gazeux résiduel (15) y subisse une augmentation de pression, le flux gazeux résiduel (15) sous pression augmentée étant introduit dans le système de réacteur de méthanol (4) afin d'y être partiellement transformé en méthanol (1), un flux de récupération (6) qui est constitué d'un gaz résiduel (16a), lequel n'a pas réagi et est issu du premier étage de réacteur (21a), étant introduit dans un système de récupération d'hydrogène (5) afin d'obtenir un flux de H à recycler (7), le flux de H à recycler (7) comportant de l'hydrogène n'ayant pas réagi qui est issu du gaz résiduel (16a) n'ayant pas réagi, l'hydrogène n'ayant pas réagi qui fait partie du flux de H à recycler (7) étant introduit de nouveau dans le premier étage de réacteur (21a) afin d'y être au moins partiellement transformé en méthanol (1), **caractérisé en ce que** l'hydrogène n'ayant pas réagi qui fait partie du flux de H à recycler (7) subit ensemble avec les oxydes de carbone n'ayant pas réagi, après avoir quitté le premier étage de réacteur (21a) et jusqu'à ce qu'il soit de nouveau introduit dans le premier étage de réacteur (21a), au moins partiellement une augmentation de pression qui est effectuée par le compresseur de recyclage (14).

2. Procédé selon la revendication 1, **caractérisé en ce que** le système de réacteur de méthanol (4) comprend un dispositif de séparation de méthanol (17) permettant d'obtenir le gaz résiduel (16a) n'ayant pas réagi qui est issu du premier étage de réacteur (21a) et un flux de méthanol brut (19a) qui est issu du premier étage de réacteur (21a), notamment **en ce que** le dispositif de séparation de méthanol (17) comprend un dispositif de condensation (18a) permettant d'obtenir, par condensation, le gaz résiduel (16a) n'ayant pas réagi qui est issu du premier étage de réacteur (21a) et le flux de méthanol brut (19a) qui est issu du premier étage de réacteur (21a).

3. Procédé selon les revendications 1 ou 2, **caractérisé en ce qu'**une partie du flux gazeux résiduel (15) sous pression augmentée est dérivée vers un branchement et introduite dans le système de réacteur de gaz de synthèse (13), préférentiellement **en ce que** la partie du flux gazeux résiduel (15) sous pression augmentée qui est dérivée vers un branchement, est introduite dans le flux de vecteur d'énergie (11).

4. Procédé selon l'une des revendications 1 à 3, **caractérisé en ce que** le système de réacteur de méthanol (4) comporte une pluralité d'étages de réacteur (21a, b) destinés à la synthèse de méthanol qui sont disposés de manière à se succéder au cours du procédé, préférentiellement **en ce que** le compresseur de recyclage (14) est disposé à manière à se trouver entre deux étages de réacteur (21a, b) au cours du procédé, notamment **en ce que** le dispositif de séparation de méthanol (17) permet d'obtenir, à partir de chacun de la pluralité d'étages de réacteur (21a, b), un gaz résiduel (16a, b) n'ayant pas réagi.

5. Procédé selon la revendication 4, **caractérisé en ce que** le flux de H à recycler (7) est introduit dans le gaz résiduel (16b) n'ayant pas réagi qui est issu d'un étage de réacteur (21b) disposé au cours du procédé en aval du premier étage de réacteur (21a), préférentiellement **en ce que** le flux de H à recycler (7) est introduit, ensemble avec le flux gazeux résiduel (15), dans le compresseur de recyclage (14) afin d'y subir une augmentation de pression.

6. Procédé selon les revendications 4 ou 5, **caractérisé en ce que** le flux gazeux résiduel (15) est obtenu à partir d'un étage de réacteur (21b) disposé au cours du procédé en aval du premier étage de réacteur (21a), notamment **en ce que** le compresseur de recyclage (14) introduit le flux gazeux résiduel (15) sous pression augmentée dans le premier étage de réacteur (21a).

7. Procédé selon la revendication 6, **caractérisé en ce que** le flux gazeux résiduel (15) est obtenu à partir d'un étage de réacteur (21b) qui est disposé de manière à être, au cours du procédé, le dernier de la pluralité d'étages de réacteur (21a, b).

8. Procédé selon l'une des revendications 1 à 7, caractérisé ce que le flux de récupération (6) est au moins partiellement dérivé, à partir du gaz résiduel (16a) n'ayant pas réagi qui est issu du premier étage de réacteur (21a), vers un branchement, de préférence encore en ce que le flux de récupération (6) est au moins partiellement dérivé de manière à ce que ledit branchement se situe au cours du procédé en amont du compresseur de recyclage (14).

9. Procédé selon la revendication 8, **caractérisé en ce que** le flux de récupération (6) du système de récupération d'hydrogène (5) est introduit sous une pression d'introduction qui est plus élevée qu'une pression de gaz résiduel à laquelle le flux gazeux résiduel (15) est obtenu à partir du système de réacteur de méthanol, préférentiellement **en ce que** le flux de récupération (6) est au moins partiellement dérivé à partir du flux gazeux résiduel (15) vers un branchement situé au cours du procédé en aval du compresseur de recyclage (14).

10. Procédé selon l'une des revendications 1 à 9, **caractérisé en ce que** le flux de H à recycler (7) est introduit notamment dans le flux de gaz de synthèse (2) sous pression augmentée.

11. Procédé selon l'une des revendications 1 à 10, **caractérisé en ce que**, pour obtenir le flux de gaz de synthèse (2), on introduit un flux contenant de l'oxygène (22) dans le système de réacteur de gaz de synthèse (13), notamment **en ce que** l'on obtient le flux de gaz de synthèse (2) à partir du flux de vecteur d'énergie (11) carboné en mettant en œuvre, au sein du système de réacteur de gaz de synthèse (13), un reformage autotherme ou une oxydation partielle, de préférence encore **en ce que** l'on obtient le flux contenant de l'oxygène (22) à partir d'un dispositif de liquéfaction fractionnée de l'air (23) destinée à produire un flux d'oxygène à partir de l'air ambiant, et notamment **en ce que** le flux contenant de l'oxygène (22) est sensiblement constitué d'oxygène.

12. Procédé selon l'une des revendications 1 à 11, **caractérisé en ce que** le flux de H à recycler (7) est introduit dans le flux de vecteur d'énergie (11), notamment au cours du procédé en amont du système de réacteur de gaz de synthèse (13), préférentiellement **en ce que** le système de récupération d'hydrogène (5) produit un flux de purge (8) lequel est notamment ensuite évacué en vue d'être brûlé.

13. Procédé selon l'une des revendications 1 à 12, **caractérisé en ce que** le flux de H à recycler (7) comporte une proportion molaire d'hydrogène plus élevée que le flux de récupération (6), préférentiellement **en ce que** le flux de H à recycler (7) comporte en proportion molaire d'hydrogène plus élevée que le flux de purge (8).

14. Procédé selon l'une des revendications 1 à 13, **caractérisé en ce que** le système de récupération d'hydrogène (5) comporte un dispositif d'adsorption par inversion de pression (24) permettant d'obtenir le flux de H à recycler (7) à partir du flux de récupération (6), de préférence encore **en ce que** le flux de H à recycler (7) est sensiblement constitué d'hydrogène.

15. Procédé selon l'une des revendications 1 à 14, **caractérisé en ce que** l'hydrogène n'ayant pas réagi qui fait partie du flux de récupération (6) subit, à partir du premier étage de réacteur (21a) et jusqu'à ce qu'il soit de nouveau introduit dans le premier étage de réacteur (21a), exactement une augmentation de pression ensemble avec les oxydes de carbone n'ayant pas réagi, celle-ci étant effectuée par le compresseur de recyclage (14).

16. Installation de synthèse de méthanol (1) pourvue d'un système de réacteur de gaz de synthèse (13) destiné à obtenir, à partir d'un flux de vecteur d'énergie (11) carboné, un flux de gaz de synthèse (2) comportant de l'hydrogène et des oxydes de carbone, d'un compresseur de gaz de synthèse (3) destiné à augmenter la pression du flux de gaz de synthèse (2), d'un système de réacteur de méthanol (4) comportant un premier étage de réacteur (21a), d'un dispositif de récupération de chaleur (10) destiné à récupérer de la chaleur à partir du flux de gaze de synthèse (2), d'un système de récupération d'hydrogène (5) et d'un compresseur de recyclage (14), le flux de gaz de synthèse (2) sous pression augmentée étant au moins partiellement introduit dans le premier étage de réacteur (21a) afin d'y être partiellement transformé en méthanol (1), le système de réacteur de méthanol (4) produisant un flux gazeux résiduel (15) comportant des oxydes de carbone n'ayant pas réagi, ce flux gazeux résiduel (15) étant introduit dans le compresseur de recyclage (14) afin d'augmenter la pression du flux gazeux résiduel (15), le flux gazeux résiduel (15) sous pression augmentée étant introduit dans le système de réacteur de méthanol (4) afin d'y être partiellement transformé en méthanol (1), le flux de gaz de synthèse (2) étant introduit dans le dispositif de récupération de chaleur (10) et ensuite dans le compresseur de gaz de synthèse (3), un flux de récupération (6) qui est constitué d'un gaz résiduel (16a) n'ayant pas réagi et qui est issu du premier étage de réacteur (21a) étant introduit dans le dispositif de récupération d'hydrogène (5) afin d'obtenir un flux de H à recycler (7), ce flux de H à recycler (7) comportant de l'hydrogène n'ayant pas réagi qui est issu du gaz résiduel (16a) n'ayant pas réagi, cet hydrogène n'ayant pas réagi, qui fait partie du flux de H à recycler (7), étant de nouveau introduit dans le premier étage de réacteur (21a) afin d'y être au moins partiellement transformé en méthanol (1), **caractérisée en ce que** l'hydrogène n'ayant pas réagi qui fait partie du flux de H à recycler (7) subit ensemble avec les oxydes de carbone n'ayant pas réagi, à partir du premier étage de réacteur (21a) et jusqu'à ce qu'il soit de nouveau introduit dans le premier étage de réacteur (21a), au moins partiellement une augmentation de pression qui est effectuée par le compresseur de recyclage (14).
